# EUROPEAN PATENT SPECIFICATION

(11) **EP 2 271 272 B1**
(45) Date of publication and mention of the grant of the patent: **02.04.2014**
(21) Application number: 09733399.1
(22) Date of filing: 10.04.2009
(51) Int. Cl.: A61B 17/64, A61B 17/66

(54) **APPARATUS FOR USE WITH FRACTURE TABLE TO REPOSITION BONE PORTIONS**
VORRICHTUNG ZUR VERWENDUNG BEI DER NEUPOSITIONIERUNG VON KNOCHENTEILEN AUF EINEM FRAKTURTISCH
APPAREIL POUR UNE UTILISATION AVEC UNE TABLE DE FRACTURE POUR REPOSITIONNER DES PARTIES D'OS

(30) Priority: 16.04.2008 US 45500 P
(43) Date of publication of application: 12.01.2011
(73) Proprietor: Synthes GmbH, 4436 Oberdorf (CH)
(72) Inventor: MATITYAHU, Amir, M., Los Altos CA 94022 (US)
(74) Representative: Lusuardi, Werther
(86) International application number: PCT/US2009/040212
(87) International publication number: WO 2009/129142

(56) References cited:
- EP-A- 1 266 633
- WO-A-03/068082
- US-A- 1 963 897
- US-A- 2 477 562
- US-A- 2 658 507
- US-A- 4 662 365
- US-A- 5 827 283

## Description

### Priority Claim

### Field of the Invention

The present invention relates to an apparatus for repositioning portions of bone relative to each other, and more particularly to an apparatus for externally repositioning portions of bone.

### Background

The percutaneous reduction of femur fractures in a patient often involves placement of the patient in a supine position on a fracture table having a peroneal post engaging the pelvis between the two legs of the patient. The legs of the patient extend from the fracture table and are typically supported by traction devices such as footplates or other tibial or femoral traction devices. An intramedullary rod or nail is often introduced into the femoral canal to properly align fractured segments of the femur. However, femoral and other muscular forces are sometimes difficult to overcome when reducing fractured femurs, particularly for fractures that occur near the proximal end of the femur where such femoral forces are particularly strong, resulting in continued malreduction or malpositioning of the fractured segments of the femur after the intramedullary rod or nail has been placed in the bone. Such malreduction or malpositioning is exacerbated when the outer diameter of the nail or rod is smaller than the inner diameter of the femur being reduced. Current methods for attempting to minimize such malreductions or malpositioning involve using an assistant to manually engage and retain the fractured segments of the femur before and during placement of the rod or nail. A percutaneous apparatus are needed that are not highly invasive for reducing and positioning fractured segments of a femur or other bone, yet still retain such fractured segments in a desired position during insertion of an intramedullary rod or nail into the bone.

From US 2,477,562 ANDERSON an orthopedic table with a fracture reducing apparatus is known. This know apparatus includes a first and a second arm that can be coupled to the orthopedic table by means of a first and a second securement mechanism permitting a pivotable movement of the first and second arms about separate axes of rotation that are spaced apart from each other.

### Summary of the Invention

A medical apparatus has been provided that includes first and second arms coupled to a fracture table. A first attachment mechanism is carried by the first arm for securing the first arm to a first portion of a bone of a mammalian body and a second attachment mechanism is carried by the second arm for securing the second arm to a second portion of the bone. The first and second arms permit respective adjustment of the first and second portions of the bone so as to desirably align the first and second portions of the bone relative to each other.

### Brief Description of the Drawings

Fig. 1 is a side view of a fracture table with a medical apparatus for repositioning bone portions according to an exemplary embodiment of the present invention;
Fig. 2 is a perspective view of the medical apparatus of Fig. 1;
Fig. 3 is a side view of a peroneal post of the medical apparatus of Fig. 1;
Fig. 4 is a top view of the peroneal post of Fig. 3 taken along the line 4-4 of Fig. 2;
Fig. 5 is an enlarged side view of the peroneal post of Fig. 3 taken along the line 5-5;
Fig. 6 is an exploded view of segments of the first arm of Fig. 2;
Fig. 7 is a side view of a segment of the first arm of Fig. 6 taken along the line 7-7;
Fig. 8 is a perspective view of a screw lock assembly of the medical apparatus of Fig. 2;
Fig. 9 is an exploded view of segments of the second arm of Fig. 2;
Fig. 10 is a bottom view of a segment of the second arm of Fig. 9 taken along the line 10-10;
Fig. 11 is an exploded view of proximal segments of the first and second arms and other components of the medical apparatus of Fig. 2 for mounting on the peroneal post of Fig. 3;
Fig. 12 is a bottom view of a washer of the medical apparatus of Fig. 2;
Fig. 13 is an exploded view of a portion of the adjustment mechanism relative to a segment of an arm of the medical apparatus of Fig. 2;
Fig. 14 is a plan view of a portion of the adjustment mechanism of Fig. 13 taken along the line 14-14;
Fig. 15 is a side view of the portion of the adjustment mechanism of Fig. 14 taken along the line 15-15;
Fig. 16 is a first side view of another portion of the adjustment mechanism of Fig. 13 taken along the line 16-16;
Fig. 17 is a second side view of the portion of the adjustment mechanism of Fig. 16 taken along the line 17-17;
Fig. 18 is a side view of an attachment mechanism of the medical apparatus of Fig. 2;
Fig. 19 is a side view of an attachment mechanism according to another embodiment of the present invention;
Fig. 20 is a plan view of a kit of the present invention;
Fig. 21 is a side view of the medical apparatus of Fig. 2 coupled to first and second portions of a segmented bone in a first position;
Fig. 22 is a top view of the medical apparatus of Fig. 2 coupled to first and second portions of a segmented bone in the first position of Fig. 21 taken along the line 22-22;
Fig. 23 is a side view of the medical apparatus of Fig. 2 coupled to first and second portions of a segmented bone repositioned to a second position relative to each other;
Fig. 24 is a top view of the medical apparatus of Fig. 2 coupled to first and second portions of a segmented bone in the second position of Fig. 23 taken along the line 24-24;
Fig. 25 is a side view of another embodiment of the medical apparatus of the present invention coupled to first and second portions of a segmented bone; and
Fig. 26 is a top plan view of the medical apparatus of Fig. 25 coupled to first arid second portions of a segmented bone taken along the line 26-26.

### Detailed Description

The present invention is direction to a medical apparatus to aid in bone fixation of a segmented bone such as, for example a fractured or osteotomised bone, as those skilled in the art will understand. An exemplary medical apparatus 31 according to the present invention is configured for attachment to an operating table on which a patient rests. The medical device 31 comprises first and second arms 53, 54 extending laterally from a central post 51. The arms 53, 54 are selectively positionable to permit alignment and subsequent attachment to first and second pins 251, 261 attached to target portions of each of two bone fragments. Once engaged, manipulation of the arms 53, 54 permits realignment of the bone fragments. The medical apparatus 31 according to the present invention thus facilitates proper alignment of fragments of a fractured bone and further maintains a stabilized position thereof until a bone fixation procedure has been performed, as those skilled in the art will understand. It is noted that although embodiments of the present invention will be described with respect to a particular bone, the present invention may be employed with a bone fixation procedure for any bone in a living body without deviating from the scope of the present invention. As used in this application, the term bottom refers to a direction approaching an operating table on which the medical apparatus is mounted and the term top refers to a direction facing away from the operating table. Furthermore, the term proximal, as used in this application refers to a direction radially approaching the central post 51 while the term distal refers to a direction extending radially outward therefrom.

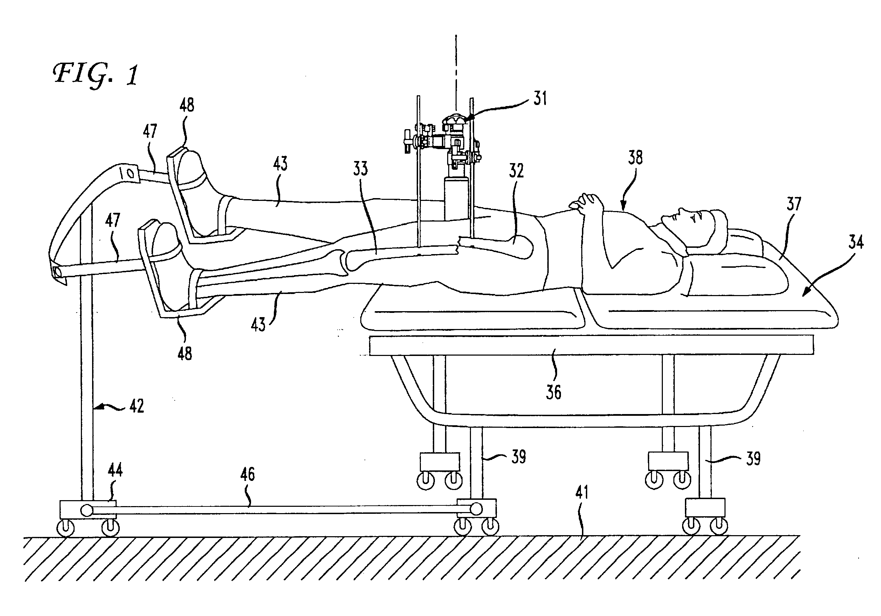

As shows in Figs. 1 - 26, the medical apparatus 31 according to an exemplary embodiment of the present invention is configured to be mountable to a conventional fracture table 34, having a top layer or bed 36 provided with an upper surface 37 for receiving a patient 38 in a supine position, in place of a peroneal post. The fracture table 34 has first and second legs 39 maintaining the table upright on a support surface 41. A conventional traction apparatus 42 is typically provided for supporting the legs 43 of the patient, as those skilled in the art will understand. The traction apparatus 42 has a base 44 which engages the support surface 41 and is preferably coupled to the fracture table 34 by any suitable means such as a bar 46 extending between base 44 and one of legs 39 of the fracture table. The traction apparatus 42 can have first and second arms 47 for supporting respective first and second foot pieces 48 capable of receiving respective feet of the patient. Medical apparatus 31 mounts to an end of the upper surface 37 of the fracture table 34 adjacent the traction apparatus 42 and is preferably positioned adjacent a pelvis of the patient 32.

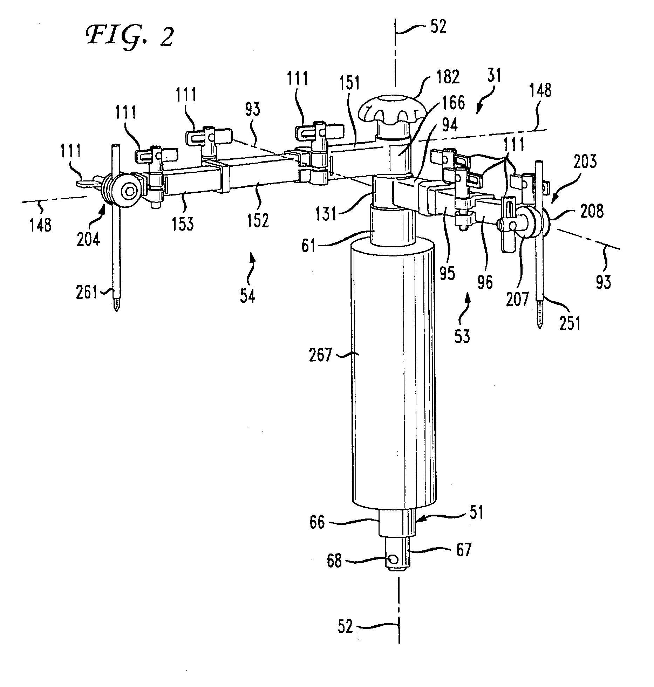

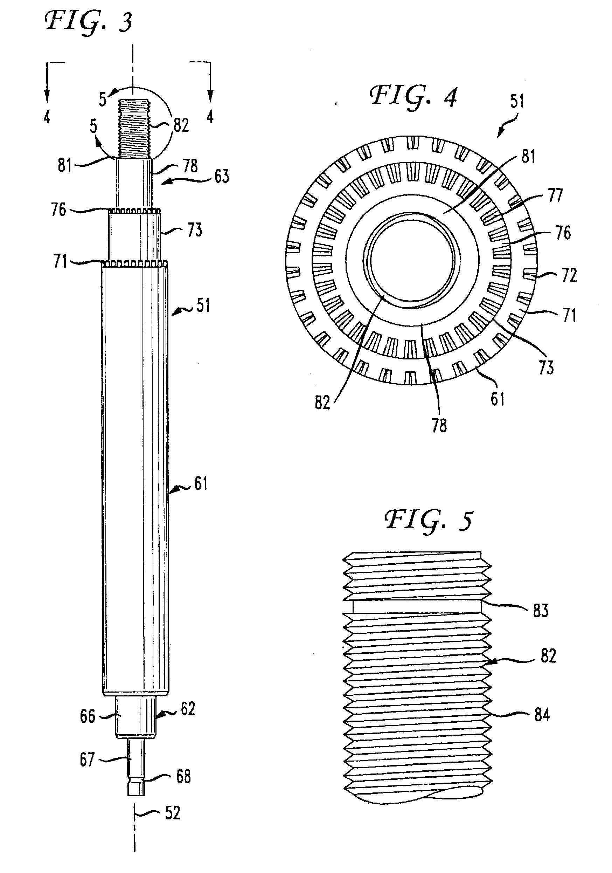

As shown in greater detail in Fig. 2, the central post 51 of the medical apparatus 31 defines a central longitudinal axis 52. The first arm 53 and second arm 54 are pivotably coupled to the top portion of post 51 for pivotable movement about the axis 52. It is noted that although the exemplary embodiment of the present invention comprises two arms 53, 54 any number of arms may be employed without deviating from the scope of the present invention. Specifically, the number of arms 53, 54 may be chosen to accommodate a number of bone fragments to be repositioned. The post 51 may be made from any suitable material such as stainless steel, aluminum, plastic, composites or radiolucent or radio-opaque material and has a length ranging from 10 to 100 centimeters, preferably approximately 20 to 60 centimeters and more preferably approximately 42.5 centimeters. As shown in Fig. 3, the post 51 includes an elongated central portion 61, a bottom portion 62 and a top portion 63. The bottom portion 62 can be of any suitable or conventional type for mounting to fracture table 34 and, in one embodiment, necks down from the central portion 61 to a first cylindrical extension 66 and, subsequently, a second cylindrical extension 67 having a smaller diameter than the first extension 66. In an operative position when the medical apparatus is mounted to the table 34, the first and second extensions 66, 67 comprise respective bores (not shown) in communication with the upper surface 37 of fracture table 34. A transverse bore 68 is provided in second extension 67 for receiving a capture pin (not shown) to secure the bottom portion 62 to the table 34.

The central portion 61 of the central post 51 has a length ranging from 5 to 80 centimeters, preferably approximately 20 to 40 centimeters and more preferably approximately 27 centimeters and extends upwardly to a first shoulder 71 having a plurality of circumferentially spaced-apart upstanding registration elements in the form of teeth 72 provided thereon, as shown in Fig. 4. A first cylindrical neck 73 extends upwardly from the first shoulder 71 by a distance ranging from 5 to 200 millimeters, preferably 20 to 40 millimeters and more preferably approximately 30 millimeters and has a diameter ranging from 10 to 100 millimeters and preferably approximately 30 millimeters. The first neck 73 terminates at a second annular shoulder 76 having a plurality of circumferentially spaced-apart upstanding registration elements in the form of teeth 77 extending therearound. A second cylindrical neck 78 having a length ranging from 5 to 200 millimeters, preferably 20 to 40 millimeters and more preferably approximately 30 millimeters extends upwardly from second shoulder 76. The second neck 78 has a diameter ranging from 5 to 95 millimeters and preferably approximately 20 millimeters. The second neck 78 extends to a third annular shoulder 81 which comprises a cylindrical threaded section 82 having a diameter less than the diameter of second neck 78 extending upwardly therefrom. As shown in greater detail in Fig. 5, an annular groove 83 is provided amongst the external threads 84 of the threaded section 82.

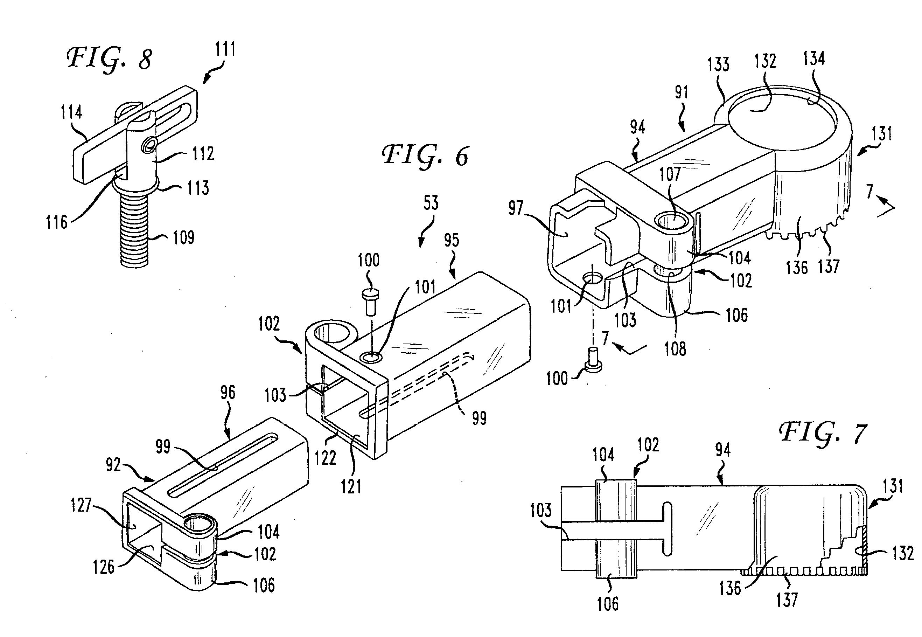

In one embodiment, the first arm 53 comprises a first proximal end portion 91 and a second distal end portion 92 and extends along a longitudinal axis 93, as shown in Figs. 2 and 6. The first arm 53 telescopes comprises three nesting arm segments that extend along axis 93, including a proximal segment 94, a central segment 95 and a distal segment 96. The proximal segment 94 includes a central bore 97 communicating with a distal opening 98 for slidably receiving the central segment 95 therein. The tubular segments of first arm 53 are formed of any suitable material such as stainless steel and are sized and shaped to preclude rotation relative to each other. As shown in Fig. 6, extension and retraction of the distal segment 96 within the central segment 95 is guided and limited by an elongated slot 99 provided in the side wall of the distal segment 96 and a screw 100 which threadably engages a threaded bore 101 provided in the corresponding side wall of the central segment 95. The threaded distal end of the screw 100 slidably engaged the slot 99 and engages an end thereof when the distal segment 96 is in a fully recessed position relative to the central segment 95.

The first arm 53 further comprises a clamping mechanism 102 at a distal end of the central segment 95 for selectively securing the central segment 95 within the proximal segment 94. In one embodiment, the proximal segment 94 has a slot 103 extending longitudinally along a side wall thereof to permit contraction of the proximal segment 94 about the central segment 95. The clamping mechanism 102 includes a first bossed element 104 on one side of slot 103 and a second bossed element 106 on the other side of the slot. The first bossed element 104 has a non-threaded bore 107 and the second bossed element 106 has a threaded bore 108 for receiving a threaded shaft 109 of a screw 111 made from any suitable material such as stainless steel. Head 112 of screw 111 has a shoulder 113 for engaging the first bossed element 104 and urging the first bossed element 104 towards the second bossed element 106 as the threaded shaft element 109 threadably engages the bore 108. In one preferred embodiment, screw head 112 includes a transversely-adjustable slide element 114 which travels within a transverse slot 116 of the screw head 112 and is engageable by the fingers of the operator for tightening and loosening screw 111 within the clamping mechanism 102. Slidable element 114 provides screw 111 with an adjustable torque arm.

Central segment 95 slidably receives distal segment 96 in a manner similar to which the proximal segment 94 slidably receives the central segment 95. In this regard, the central segment 95 includes a central bore 121 having a distal opening 122 though which the distal segment 96 longitudinally travels. Proximal and distal travel of distal segment 96 within central segment 95 is limited by the limiting mechanism of slot 99 provided, for example, in one of the side walls of the distal segment 96 and a screw 100 that extends through a threaded bore 101 provided in the corresponding side wall of the central segment 95 and which extends further into the slot 99. Central segment 95 is provided with a slot 103 and a clamping mechanism 102 that includes a screw 111 for selectively locking the distal segment 96 at a desired position within the central segment 95.

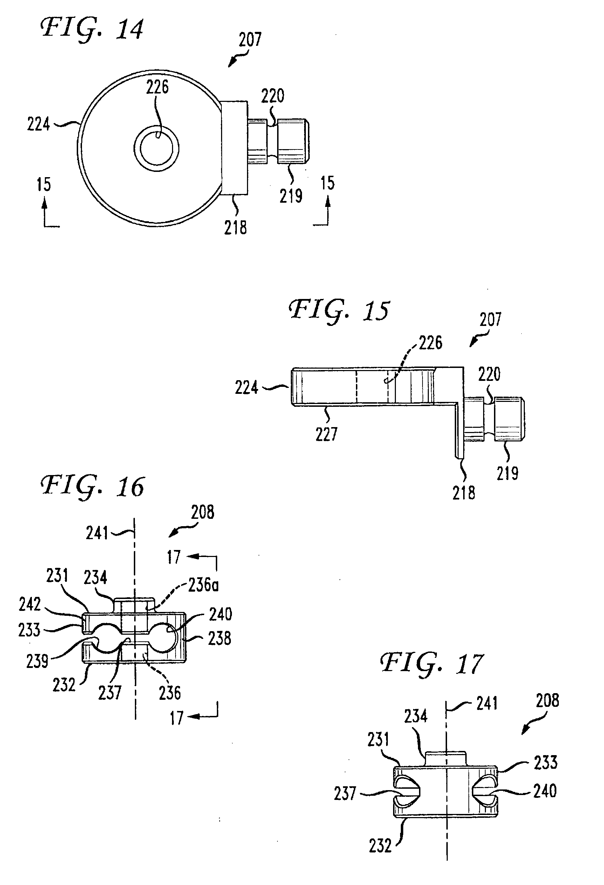

An annular first collar 131 is secured to first or proximal end portion 91 of the first arm for pivotably coupling the arm to post 51. The first collar 131 and the central post 51 are included within the first securement mechanism of the medical apparatus 31 for coupling the proximal end portion 91 of the first arm 53 to the fracture table 34. The collar 131 is provided with an internal bore 132 extending perpendicular to arm axis 93. An inwardly-extending flange 133 extends around a top surface of the first collar 131, forming an upper circular opening 134. An annular rim 136 forms the bottom of the first collar 131 at the lower opening of the internal bore 132. The rim 136 is provided with a plurality of circumferentially spaced-apart registration elements in the form of depending teeth 137. The first collar 131 is sized to extend around first neck 73 of the central post 51 with the upper opening 134 extending around a second shoulder 76 of the central post 51. Teeth 137 of the collar 131 are radially dimensioned to incrementally register with upstanding teeth 72 on the first shoulder 71 for pivotably locking the first arm 53 in a desired orientation relative to central post 51. An annular space (not shown) is provided between the first collar 131 and the first neck 73 for receiving a coil spring 138 having a lower end which seats on the first shoulder 71 radially inward of the teeth 72 with an upper end seated against the inside of the collar flange 133, as shown in greater detail in Fig. 17.

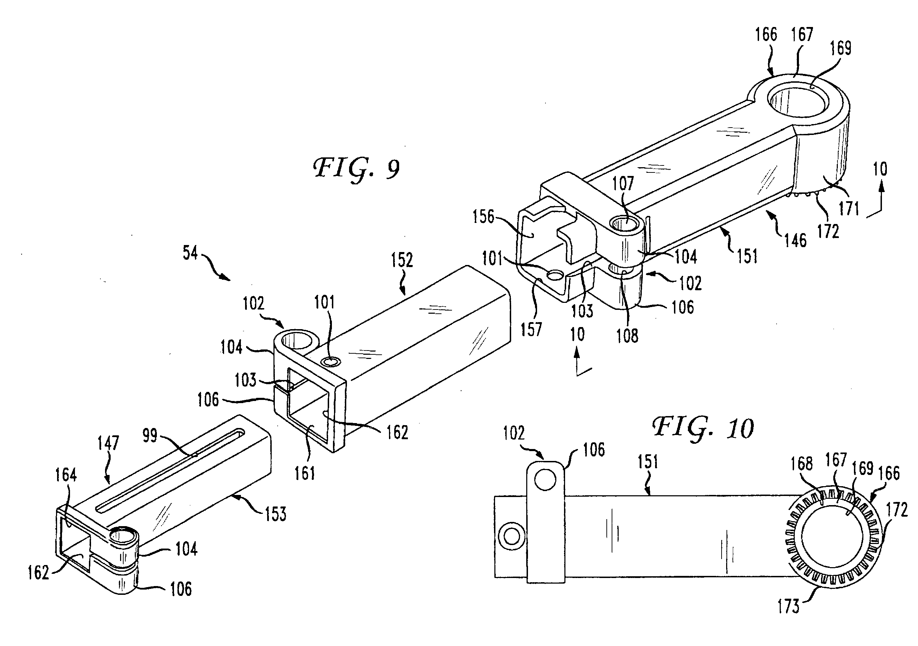

The second arm 54 has a construction substantially similar to the first arm 53. In this regard, the second arm 54, as shown in Figs. 9 and 10, has a proximal end portion 146 and a distal end portion 147 and extends along a longitudinal axis 148. The segmented second arm 54 includes a proximal segment 151, a central segment 152 and a distal segment 153, each made from any suitable material such as stainless steel, with these segments nesting within one another in a manner similar to that described above with respect to the first arm 53. The tubular proximal segment 151 is proved with a central bore 156, that communicates with a distal opening 157, for slidably receiving the central segment 152. The proximal segment 151 has a clamping mechanism 102 and a slot 103 for selectively locking the central segment 152 in a desired position with respect to the proximal segment 151. The clamping mechanism 102 includes a screw 111 (not shown in Fig. 9) that extends through the non-threaded bore 107 of the first bossed element 104 and threads into the threaded bore 108 of the second bossed element 106. The tubular central segment 152 is provided with a central bore 161 having a distal opening 162 for slidably receiving the distal segment 153. Another clamping mechanism 102, which includes another screw 111 (not shown in Fig. 9), is provided at the distal end of the central segment 152 for selectively locking the distal segment 153 at a desired position within the central segment 152. The tubular distal segment 153 of the second arm 54 is provided with a central bore 163 communicating with a distal opening 164 provided at the distal end of the segment 153 adjacent to a coupling mechanism 102.

A proximal end of the proximal segment 151 is joined to a tubular second collar 166 that is substantially similar to the first collar 131 of the first arm 53. A second collar 131 and a central post 51 are included within the second securement mechanism of the apparatus 31 for coupling the proximal end portion 46 of the second arm 54 to the table 34. The collar 166 is circular in cross section and has an inwardly-extending flange 167 at the top end thereof that is flush with the top surface of the proximal segment 151. An internal bore 168 is provided in the collar 166 and communicates with a circular upper opening 169 provided in the flange 168. A circular rim 171 extends around the bottom of the collar 166 and is provided with a plurality of circumferentially spaced-apart depending registration elements in the form of teeth 172 sized and spaced to cooperatively engage upstanding teeth 77 on the second shoulder 76 of the post 51 in the same manner discussed above with respect to the collar 131 and the teeth 72 on the first shoulder 71. The teeth 172 are spaced radially inward of the outer edge of the rim 171, which has an annular lower surface 173 that extends radially outside the teeth and is substantially planar. As such, the second collar 166 is radially sized so that the teeth 172 thereof engage the teeth 77 of the post 51. The upper opening 169 is radially sized to extend around the second neck 78 of the post 51 and the annular wall forming the second collar 166 is spaced from the outer cylindrical surface of the second neck 78 to provide an annular space between the collar 166 and the neck 78 for receiving a second coil spring 174, as shown in Figs. 3, 4, 10 and 17. The second coil spring 174 has a bottom end which engages the second shoulder 76 radially inside the teeth 77 and a top end which engages the bottom surface of the flange 167 of the second collar 166. A tubular distal segment 96 of the second arm 54 is provided with a central bore 126 which communicates with a distal opening 127 provided at the distal end of the segment 96 adjacent to the clamping mechanism 102.

First and second arms 53 and 54 may be of substantially the same length, but in a preferred embodiment, one of the arms is shorter than the other. In this regard, when the apparatus 31 is used to treat a fractured femur, the shorter arm is particularly suited for use with an attachment mechanism coupled to the proximal portion of the femur (i.e., the portion of the femur closer to the post 51), while the longer arm is particularly suited for use with an attachment mechanism coupled to the distal portion of the femur (i.e., the portion further from the post 51), as those skilled in the art will understand. When the shorter arm 53 or 54 is contracted, the respective distal segment 96 or 153 is fully retracted into its respective central segment 95 or 152, which is further fully retracted into the proximal segment 94 or 1 1. In this position, the shorter arm has a length ranging from 6 to 30 centimeters and preferably approximately 8 centimeters. When the shorter arm is fully extended, as described in greater detail earlier, the arm has a length ranging from 12 to 60 centimeters and preferably approximately 20 centimeters. When the longer arm 53 or 54 is contracted, such arm has a length ranging from 26 to 40 centimeters and preferably approximately 30 centimeters. When the longer arm is fully extended, such arm has a length ranging from 26 to 80 centimeters and preferably approximately 40 centimeters.

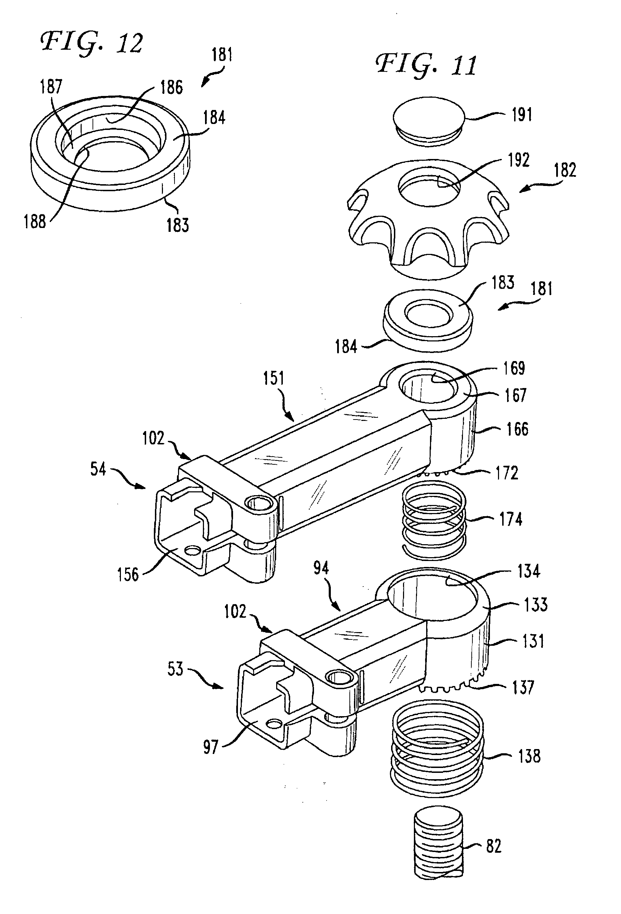

A washer 181 and a knob 182 are provided in the apparatus 31 for retaining the first collar 131 of the first ann 53 and the second collar 166 of the second arm 154 on the central post 51. As shown in Figs. 11 and 12, the washer 181 is annular in shape and has a top planar surface 183 and a bottom planar surface 184. An annular opening 186 is provided in the bottom surface 184 and extends to an inwardly-extending flange 187 forming a top opening 188 at the top surface 183. Washer 181 sits atop a second neck 78, a third shoulder 81 of which extends into the annular opening 186 to abut the flange 187. Knob 182 is provided with an internally-threaded bore (not shown) for cooperatively engaging the threaded section 82 at the top of the central post 51. When tightened on the central post 51, the knob 182 urges washer 181 against second collar 166 to rotatably lock the depending teeth 172 of the second collar with upstanding teeth 77 on the second shoulder 76. The second collar 166 is longitudinally sized so that as its depending teeth 172 seat with the upstanding teeth 77 on the second shoulder and the lower planar surface 173 thereof engages the top of first collar 131 and urges the first collar downwardly on post 51 and thus rotatably lock depending teeth 137 of the first collar 131 with upstanding teeth 72 on first shoulder 71. A cap 191 cooperatively engages an opening 192 at the top of knob 182.

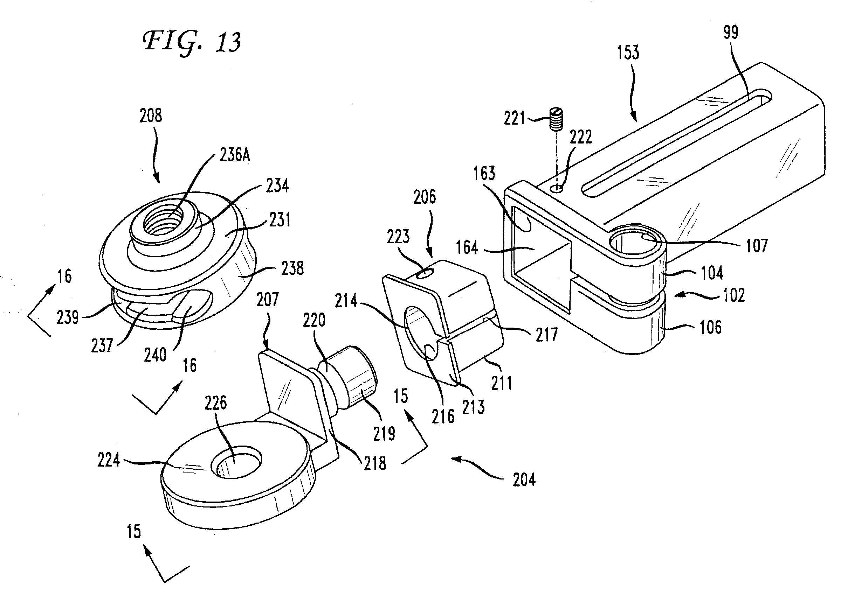

A first attachment mechanism is carried by the distal end portion or free end 92 of the first arm 53 for securing the first arm 53 to one of the bone portions 32 and 33 and a second attachment mechanism is carried by the distal end portion or free end 147 of the second arm 54 for securing the second arm 54 to the other one of the bone portions 32 and 33. In this regard, a first adjustment mechanism 203 is provided for coupling the first attachment mechanism to the distal end portion 92 of the first arm 53, and a second adjustment mechanism 204 is provided for coupling the second attachment mechanism to the distal end portion 147 of the second arm 54. The first and second adjustment mechanisms 203 and 204 of this embodiment are substantially identical and include an insert 206, a pivot element 207, a disk 208 and a screw 111, as shown in Figs. 13 - 17. The insert 206, the pivot element 207 and the disk 208 may each be made from any suitable material such as stainless steel. Fig. 13 illustrates the second adjustment mechanism 204 relative to the distal segment 153 of the second arm 54. The insert 206 includes a neck 211 sized and shaped to non-rotatably fit within the central bore 163 of the distal segment 153 and is preferably rectangular in cross section. A planar flange 213 is disposed at the distal end of the neck 211 and can seat flush against the distal end of the segment 153 to limit the travel of the neck 211 into the bore 163. A circular aperture 214 is provided in the center of the flange 213 and provides the opening to a central bore or socket 216 extending through the flange 213 and the neck 211. A slot 217 extends from the socket 216 through the flange 213 and the neck 211. The insert 206 is secured within the central bore 163 by the clamping mechanism 102 provided at the distal end of the arm segment 153 which provides a compression fit around the neck 211 of the insert 206.

The pivot element 207 which allows the second pin 261 to be positioned as desired by rotation about 2 mutually perpendicular axes may be modified if desired to add a third mutually perpendicular axis of rotation to permit any desired positioning of the pin 261 as would be understood by those skilled in the art. The pivot element 207, as shown most clearly in Figs. 13 - 15, includes a planar end wall 218 and a pin element or journal 219 extending perpendicularly from the wall 218. The journal 219 is cylindrical in shape and provided with an annular groove 220 on the cylindrical surface thereof. The journal 219 is radially sized and shaped to rotatably fit within the socket 216 of the insert 206 and can be rotatably locked relative to the insert 206 by means of the clamping mechanism 102. Specifically in this regard, the urging together of the first and second bossed elements 104 and 106 of the clamping mechanism by the screw 111 disposed within the bores 107 and 108 causes the neck 211 of the insert 206 to compress about the journal 219 and frictionally restrict rotation of the journal 219 within the socket 216. The slot 217 in the insert 206 permits the insert 206 to contract about the journal 219 under the compression force of the clamping mechanism 102. The journal 219 may be locked within the insert 206 by any suitable means such as a pin 221 that is press fit, screwed or otherwise secured within a bore 222 in the distal end of the arm segment 153 and extends through a bore 223 provided in neck 211 of the insert 206 into the groove 220 of the journal 219. A planar base 224 extends from the wall 211 of the pivot element 207 in a direction opposite the journal 219. The base 224 is preferably circular in shape and is provided with a bore 226 extending through the center thereof and opening on to a planar surface 227 which abuts the end wall 218.

The disk 208 is formed with first and second opposite planar surfaces 231 and 232 and a cylindrical surface 233 extending between the planar surfaces 231 and 232. An upstanding cylindrical hub 234 protrudes perpendicularly from the center of the first planar surface 231. A bore 236 extends through the center of the hub 234 to the second planar surface 232. A slot 237 extends perpendicularly from the cylindrical surface 233 parallel to the surfaces 231 and 232, through at least half of the disk 208 to a side wall 238. The slot 237 is preferably midway between the surfaces 231 and 232. The portion 236a of the bore 236 extending through the hub 234 to slot 237 is threaded. A first bore 239 extends through the cylindrical surface 233 parallel to the surfaces 231 and 232 adjacent to the open end of the slot 237. An optional second bore 240 extends parallel to the first bore 239 near the end of the slot 237 adjacent to the side wall 238. The first and second bores 239 and 240 are preferably symmetrically disposed about the centerline or axis 241 of the disk 208. The section of the disk 208 that includes the hub 234 and the portion of the disk between the first planar surface 231 and the slot 237 serves as a clamping portion 242 that hinges about the side wall 238 relative to the portion of the disk 208 between the slot 237 and the second planar surface 232.

In use, the disk 208 is placed in juxtaposition to the base 224 of the insert 206, with the second planar surface 232 of the disk 208 disposed against the planar surface 227 of the base 224. The disk 208 is centered on the base 224 so that the bore 236 of the disk 208 is centered on the bore 226 of the base 224. The threaded shaft 109 of a pin element or screw, for example screw 111, extends through the bore 226 into the disk 208 threadedly engaging the threaded portion 236a of the bore 236. When the screw 111 is not fully tightened relative to the base 224, the disk 208 can rotate about its center line 241 relative to the base 224. Once the shoulder 113 of the screw 111 abuts the base 224 and the screw 111 is further rotated relative to the insert 208, the clamping portion 242 of the disk 208 is urged by the screw 111 toward the base 224. The optional second bore 240 enhances pivoting of the clamping portion of 242 relative to the remainder of disk 208 by providing a relief within the disk 208 so as to minimize stress at the side wall 238, the hinge point of the clamping portion 242

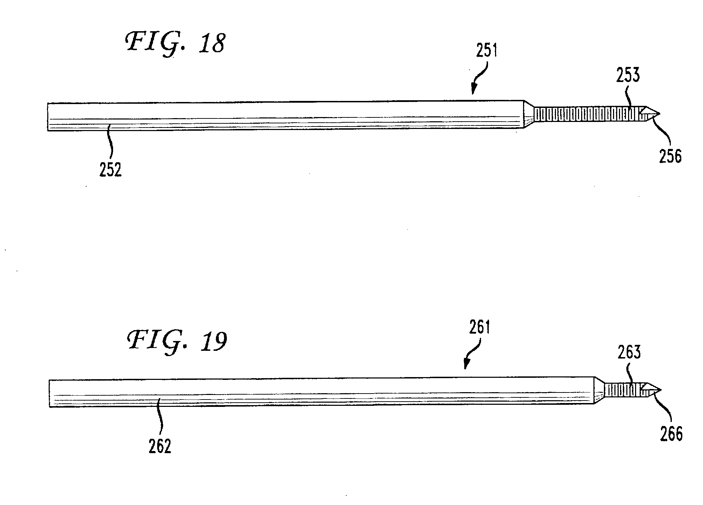

Each of the first and second adjustment mechanisms 203 and 204 can be in the form of an elongate pin, for example as shown in Figs. 18 and 19. The first pin 251, shown in Fig. 18, is formed as an elongate cylindrical body made from any suitable material such as stainless steel and having a non-threaded proximal portion 252 and a distal portion 253 that is threaded. The threaded distal portion 253 ends at a pointed tip 256. A second pin 261, illustrated in Fig. 19, is similar to the first pin 251 and is as an elongate cylindrical body made from any suitable material such as stainless steel. The second pin 261 includes a non-threaded proximal portion 262 and a threaded distal portion 263. The threaded distal portion 263 of the second pin 261 is shorter in length than the threaded distal portion 253 of the first pin 251 and is provided with a pointed tip 266. Each of the pins can have a length ranging from five to 100 centimeters and preferably approximately 25 centimeters. The non-threaded proximal portions of the pins 251 and 261 can range in length from five to 40 centimeters and the non-threaded distal portions of the pins can range in length from five to 100 millimeters.

The disk 208 is configured to receive the respective first or second adjustment mechanism 203 or 204, for example first or second pin 251 or 261. In this regard, at least the first bore 239 of the disk 208 is shaped to receive the proximal portion of the respective pin. When utilizing the first pin 251, the proximal portion 252 of the pin is inserted into the bore 239. As the threaded shaft 109 of the respective screw 111 is advanced through the threaded bore 236a of the disk 208, the slot 237 permits the disk 208 to clamp about the shaft of the pin 251 to fixedly secure the second pin 252 to the disk 208 of the second adjustment mechanism 204.

As shown in Fig. 2, the apparatus 31 further includes a tubular cushion element 267 formed from any suitable deformable material extending around the portion of the post 51 engaged by the patient.

Although medical apparatus 31 has been illustrated and described for use with attachment mechanisms in the form of elongate pins, it will be understood by those skilled in the art that any suitable attachment mechanism for coupling to a portion of a bone of a mammalian body can be utilized therewith. In this regard, the first attachment mechanism, for example for use with first arm 53, can alternatively be in the form of a wire, a screw, a hook or a clamp, each of any suitable type. Further, the second attachment mechanism, for example for use with second arm 54, can alternatively can be in a form of a wire, a crew, a hook or a clamp, each of any suitable type. The first attachment mechanism can be of a different type then the second attachment mechanism.

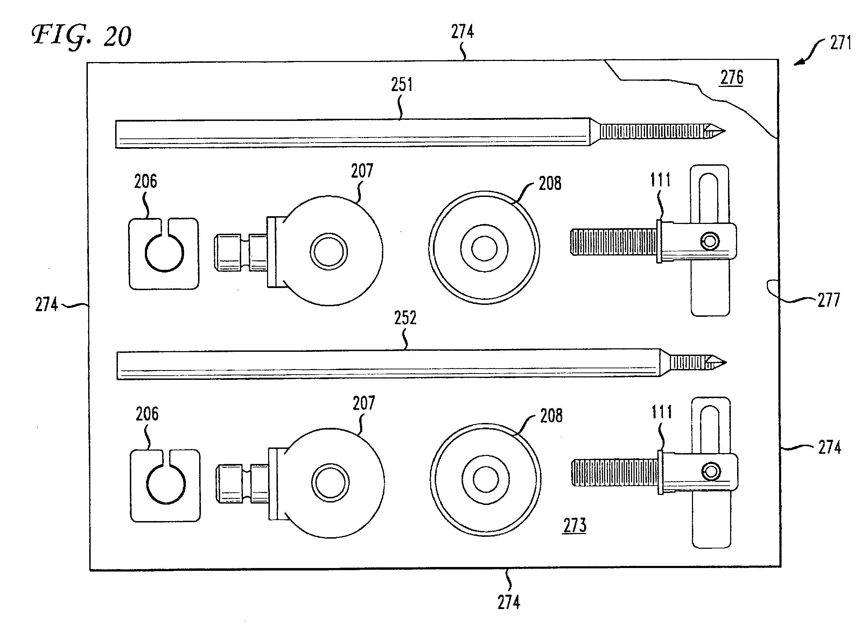

Fig. 20 depicts a kit 271 containing portions of the apparatus 31 and one or more attachment mechanisms. The kit 271 includes a conventional package 272 formed from the base 273, four side walls 274 and a top wall 276. The kit 271 is shown with a portion of the top wall 276 removed to reveal an internal cavity 277. The components of the kit 271 include the pivot element 207 and the disk 208 and at least one attachment mechanism, shown in the form of a first pin 251. Additional optional components of the kit 271 include any or all of one or more additional pivot elements 207, one or more additional disks 208, one or more inserts 207, one or more screws 111 and one or more additional attachment mechanisms. In the illustrated embodiment of the kit 271, a second pivot element 207, a second disk 208, a second pin 261 and two inserts 206 are additionally included.

In operation and use of the apparatus 31 and the kit 271, a patient 38 is placed on an upper surface 37 of a fracture table 34 with the ends of legs 43 secured to respective arms 47 of the traction apparatus 42 by any suitable means, such as by coupling the feet of the patient into respective foot pieces 48. The bottom portion 62 of the central post 51 is secured to fracture table 34 by any suitable means, as discussed above, between legs 43 of the patient, and the patient's pelvis is urged against the cushion 267 of the apparatus 31.

In one suitable procedure, threaded section 254 of the first pin 251 is inserted into a first bone portion 32 and the threaded section 264 of the second pin 261 is threaded into a second portion 33 of the target bone to be treated. In one preferred procedure, the first and second pins 251 and 261 are introduced into the first and second bone portions 32 and 33 of a femur. The relatively long threaded section 254 of the first pin 251 is particularly desirous for placement in the compact bone of the upper portion of the femur, while the relatively short threaded section 264 of the second pin 261 is particularly advantageous for placement within the compact bone of the central portion or the lower portion of the femur. Preferably, such threaded sections are longitudinally sized so as to not extend fully through the respective bone portion into which the pin is secured.

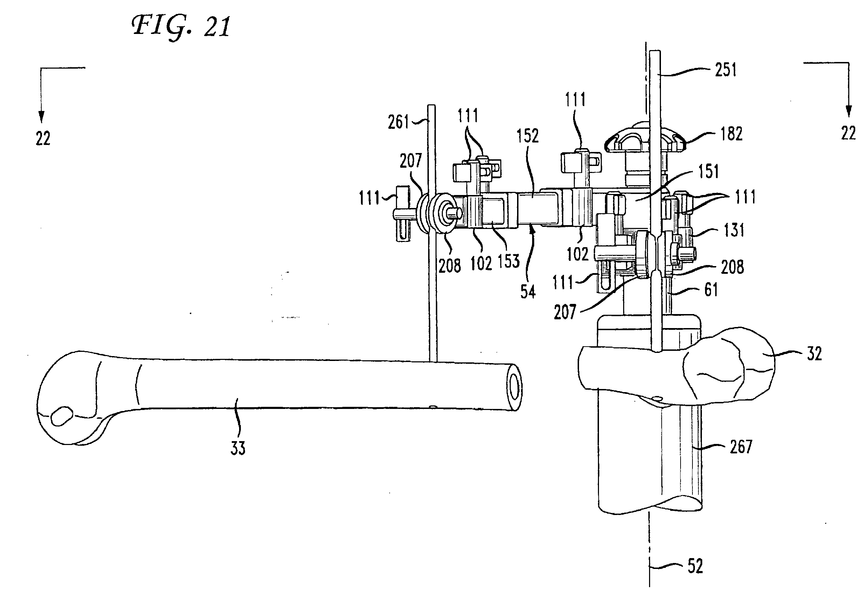

As shown in Figs. 2 and 21, once first and second pins, or other suitable attachment mechanisms, have been secured to first and second bone portions 32 and 33, the pins 251 and 261 are secured to appropriate arms 53 and 54 of medical apparatus 31 so that the first and second bone portions are coupled to fracture table 34. In one preferred procedure, first pin 251 secured to the upper portion of the femur is coupled to disk 208 of the first adjustment mechanism 203 attached to the distal end of first arm 53. Second pin 261 is secured to the second adjustment mechanism 254 coupled to the distal end portion of second arm 54. In these steps, first and second arms 53 and 54 are desirably positioned relative to the pins 251 and 261 by untightening knob 182 of top post 51 so as to permit first and second arms 53 and 54 to be pivoted about common axis 52 of the post to the desired positions relative to fracture table 34. Each of the arms 53 and 54 is adjusted to a desired length by moving or translating the central and distal segments of the arm relative to the respective proximal segment of the arm and thereafter securing the central and distal segments by tightening the screws 111 at the distal ends of the proximal segment and the central segment. The pins 251 and 261 are secured to respective adjustment mechanisms 203 and 204 by either inserting the proximal portion of the respective pin through the first bore 239 of the respective disk 208 or loosening the screw 111 of the respective adjustment mechanism sufficiently so that such proximal portion can be slide through the opening of slot 237 and into the first bore 239 of the respective disk 208. Screw 111 at the distal end of the respective distal segment can be loosened to permit the pivot element 207 to pivot about the respective longitudinal axis 93 or 148, and thus pivotably align the first bore 239 in a first axis with the pin, and thereafter tightened. The screw 111 extending through respective pivot element 207 and into the respective disk 208 can be loosened to permit the disk 208 to pivot about its center line 241 relative to pivot element 207, and thus pivotable align the first bore 239 in a second axis orthogonal to such first axis with the pin, and thereafter tightened to lock the disk in its desired position relative to the pivot element 207. In this regard, as the screw 111 through the disk 208 causes the clamping portion 242 to tighten about the pin so as to fixedly secure the pin within the disk 208, the disk is urged against base 224 so that the frictionally engagement between surface 227 of the base 224 and surface 232 of the disk 208 preclude rotation of the disk relative to the base. As such, each of adjustment mechanisms 203 and 204 permits the respective pin to pivoted about a first axis extending along a centerline of the respective arm and a second axis extending perpendicular to such first axis, and also to be translated along such first axis.

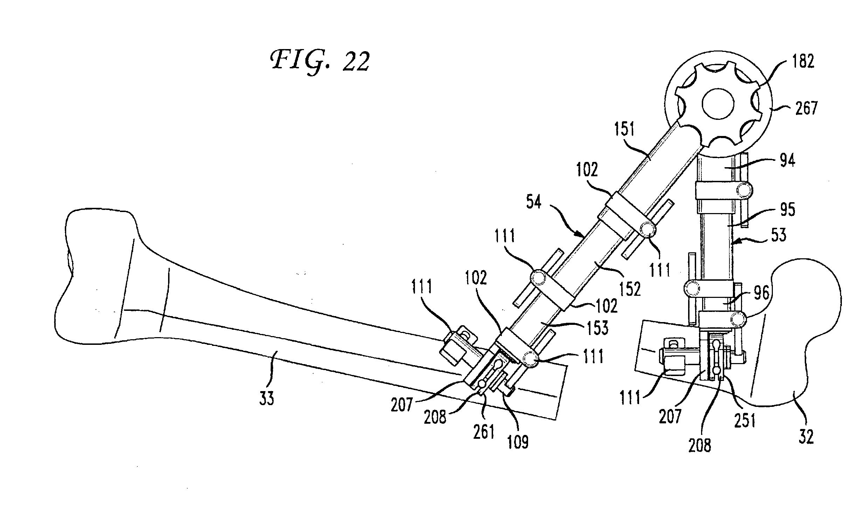

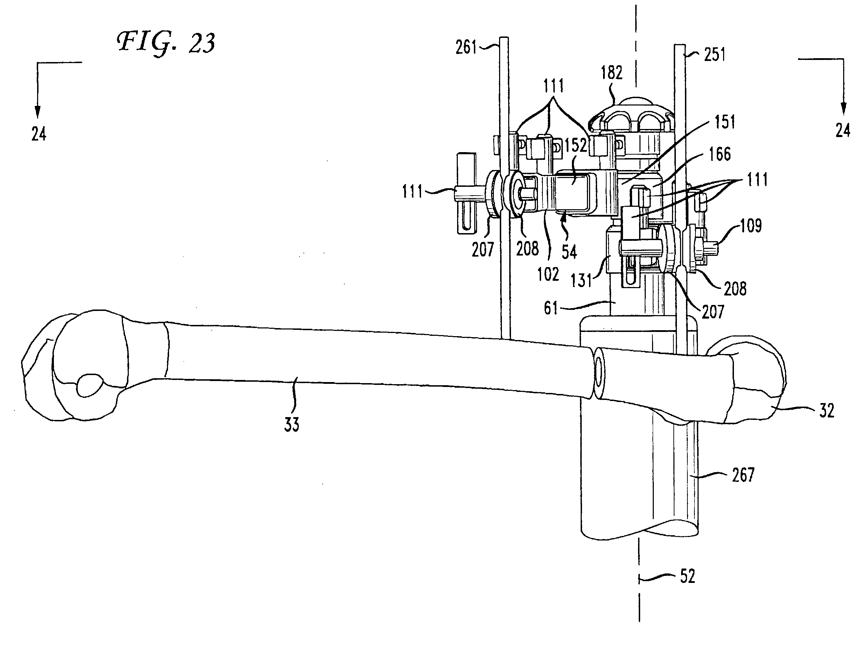

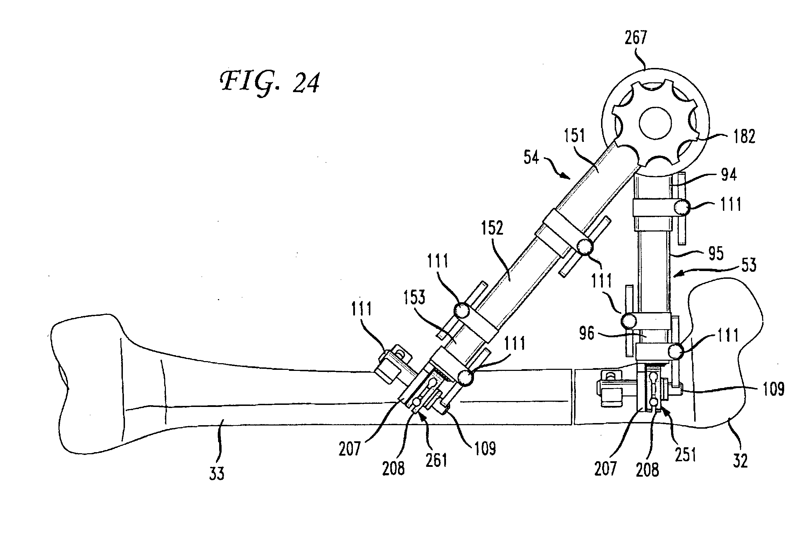

Once first and second pins 251 and 261 are secured to respective first and second arms 53 and 54, the arms can be moved relative to each other and to fracture table 34 so as to desirably reposition first and second bone portions 32 and 33 relative to each other. When moving bone portions 32 and 33, the appropriate screws 111 on first and second arms 53 and 54 and the knob 182 can be loosened or tightened to reposition the pins 251 and 261, and thus bone portions 31 and 32, relative to the fracture table. Figs. 21 and 22 illustrate the second bone portion 33 in a first position spaced posteriorly, medially and longitudinally relatively to the first bone portion 32. In Fig. 23, it can be seen that the second bone portion 33 has been moved anteriorly relative to the first bone portion 32 by retracting second pin 261 within first bore 239 of the second attachment mechanism 204. Figs. 23 and 24 further illustrate that the second bone portion 33 has been reduced, or moved longitudinally, relative to the first bone portion 32 by pivoting the second arm 54 about longitudinal axis 52 of the post 51 towards the first arm 53. Fig. 24 further illustrates that the second bone portion has been moved medially relative to the first bone portion by retracting distal segment 153 of the second arm 54 relative to central segment 152 of arm 54 such that the arm is moved longitudinally. It is appreciated that the procedure can include pivoting one or both of pins 251 and 261 by pivoting one or both of the pivot elements 207 relative to the respective insert 206 and/or pivoting one or both of disks 208 about its centerline or axis 241 relative to the respective pivot element 207. Once the first and second bone portions 32 and 33 have been fully reduced or otherwise properly positioned relative to each other, the bone portions can be fixed to each other by any suitable means such as, for example, by the introduction of an intramedullary rod or nail through the bone portions.

Although the apparatus 31 has been described and shown as having arms coupled to a fracture table that telescope relative to each other, it is appreciated that one or both of such arms can have a plurality of segments that pivot relative to each or are otherwise moveable relative to each other so that the distal portion of such arm is translatable and/or rotatable relative to the proximal portion of such arm. Each of the arms of the apparatus of the invention can have any number of segments. In addition, the arms of the apparatus of the invention can be coupled to fracture table 34 by other than the peroneal post and be within the scope of the present invention. For example, the arms can be commonly coupled to another location on the fracture table, for example to another location on the bed 36 of the fracture table. The arms of the apparatus can also be coupled to distinct or separate locations on the fracture table, for example the first arm can be coupled to one location on the bed 36 and the second arm can be coupled to another location on the bed.

Additionally, it is appreciated that one or all of the arms of the apparatus and the post of the apparatus can be provided without teeth, that is like teeth 137 and 172 of the arms and like teeth 72 and 77 of the post, so that the pivotable adjustment of such arms relative to the post is not limited to positions dictated by registration of the teeth on the arms with the teeth on the post. In one such embodiment without teeth, shoulders 71 and 76 of the post would have planar or relatively smooth surfaces and rims 136 and 171 of the arms would have planar or relatively smooth end surfaces. It is further appreciated that a variety of other pin and socket or other mechanical arrangements can be provided for permitting an attachment mechanism to pivot about one or more pivot axes at the distal end of an arm coupled to a fracture table.

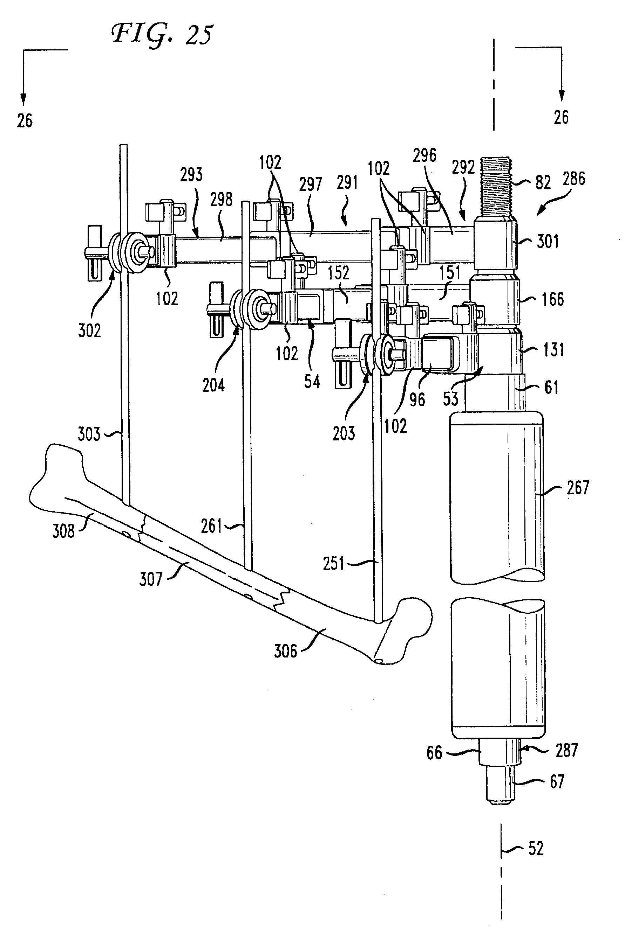

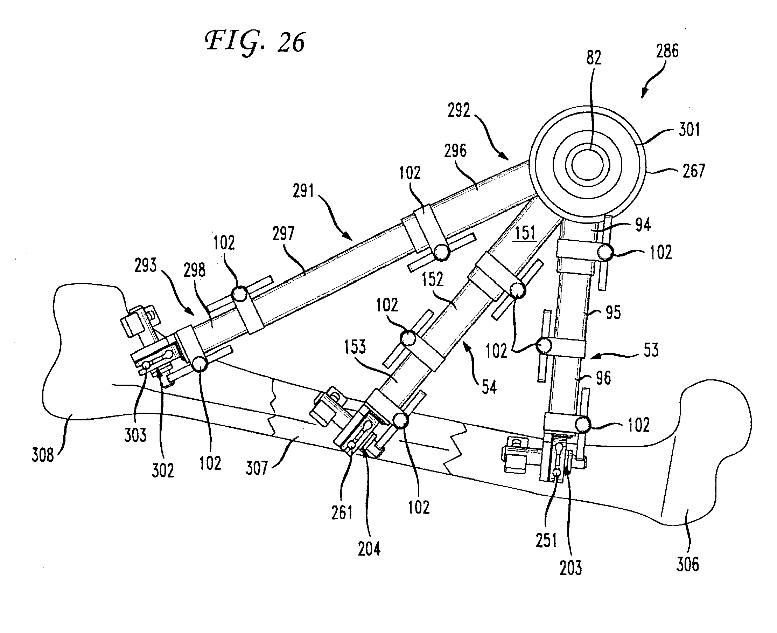

The apparatus of the present invention can have more than two arms for repositioning more than two bone portions with respect to a fracture table. Medical apparatus 286 illustrated in Figs. 25 and 26 is substantially similar to medical apparatus 31 and like reference numerals have been used to describe like components of medical apparatus 31 and 286. Medical apparatus 286 includes a post 287 substantially similar to post 51 but extended further to have a third neck (not shown) and a fourth shoulder (not shown) below threaded section 82. The apparatus 286 includes first and second arms 53 and 54 having respective first and second adjustment mechanisms 203 and 204 for coupling to respective first and second pins 251 and 261. A third arm 291 substantially similar to first and second arms 53 and 54 is provided atop second ann 54 and has a proximal end portion 292 and a distal end portion 293. Third arm 291 can be of any suitable type and is shown with a proximal segment 296, a central segment 297 that telescopes relative to the proximal segment and a distal segment 298 that telescopes relative to the central segment. A third collar 301, substantially similar to collars 131 and 166, is joined to the proximal end of segment 296 for extending around post 287 and a third coil spring (not shown), substantially similar to first and second coil springs 138 and 174, is disposed between the collar 301 and the post 287. Third collar 301 and post 51 are included within the third securement mechanism of apparatus 286 for coupling proximal end portion 292 of the third arm 291 to fracture table 34. A third adjustment mechanism 302, substantially similar to first and second adjustment mechanisms 203 and 204, is joined to the distal end portion 293 of the third arm. The third adjustment mechanism serves to couple a third attachment mechanism of any suitable type, and as shown as a third pin 303 substantially to first and second pins 251 and 261, to the third arm 291.

In operation and use, medical apparatus 286 can be used with any suitable bone, such as the femur, having a first bone portion 306, a second bone portion 307 and a third bone portion 308. The first, second and third arms of medical apparatus 86 can be joined to the respective first, second and third bone portions in the manner described above to desirably position such bone portions relative to each other and to the fracture table during any suitable procedure to treat the fractured bone.

As can be seen from the foregoing, a percutaneous apparatus that is not highly invasive has been provided for reducing and positioning fractured segments of a femur or other bone, and retaining such fractured segments in such desired position, during insertion of an intramedullary rod or nail into the bone. Since the fractured segments of the bone are coupled to the fracture table by means of respective arms coupled to the fracture table, the fractured segments do not move and instead remain in a fixed position during placement of the rod or nail and any other treatment of the bone and adjoining tissue,

Although various representative embodiments of this invention have been described above with a certain degree of particularity, those skilled in the art could make numerous alterations to the disclosed embodiments without departing from the scope of the inventive subject matter set forth in the specification and claims. All directional references (e.g., upper, lower, upward, downward, left, right, leftward, rightward, top, bottom, above, below, vertical, horizontal, clockwise, counterclockwise, x-axis, y-axis, and z-axis) are only used for identification purposes to aid the reader's understanding of the embodiments of the present invention, and do not create limitations, particularly as to the position, orientation, or use of the invention unless specifically set forth in the claims. Joinder references (e.g., attached, coupled, connected) are to be construed broadly and may include intermediate members between a connection of elements and relative movement between elements. As such, joinder references do not necessarily infer that two elements are directly.connected and in fixed relation to each other.

In some instances, components are described with reference to "ends" having a particular characteristic and/or being connected with another part. However, those skilled in the art will recognize that the present invention is not limited to components which terminate immediately beyond their points of connection with other parts. Thus, the term "end" should be interpreted broadly, in a manner that includes areas adjacent, rearward, forward of, or otherwise near the terminus of a particular element, link, component, part, member. In methodologies directly or indirectly set forth herein, various steps and operations are described in one possible order of operation, but those skilled in the art will recognize that steps and operations may be rearranged, replaced, or eliminated without necessarily departing from the scope of the present invention. It is intended that all matter contained in the above description or shown in the accompanying drawings shall be interpreted as illustrative only and not limiting. Changes in detail or structure may be made without departing from the scope of the invention as defined in the appended claims.

Although the present invention has been described with reference to preferred embodiments, it is submitted that various modifications can be made to the exemplary system without departing from the scope of the invention.

## Claims

1. A device for bone fixation, comprising:
a first arm (53) having first proximal and distal end portions (91, 92);
a second arm (54) having second proximal and distal end portions (146, 147);
a first securement mechanism (131, 51) configured to couple the first proximal end portion (91) of the first arm (53) to a fracture table;
a second securement mechanism (166, 51) configured to couple the second proximal end portion (146) of the second arm (54) to the fracture table;
a central post (51) coupling the first and second securement mechanisms (131, 51, 166, 51) to a fracture table;
a first attachment mechanism (251) carried by the first distal end portion (92) of the first arm (53) for securing the first arm (53) to a first target portion of a bone; and
a second attachment mechanism (261) carried by the second distal end portion (147) of the second arm (54) for securing the second arm (54) to a second target portion of a bone, wherein
the first and second securement mechanisms (131, 51, 166, 51) are configured to permit pivotable movement of the respective first and second arms (53, 54) relative to a fracture table about a common axis defined by the central post (51); and each of the first and second arms (53, 54) includes a plurality of arm segments (94, 95, 96, 151, 152, 153) movable relative to one another, so that
the first and second arms (53, 54) are positionable so that alignment of the first and second target portions of bone in target positions relative to one another is permitted.

2. The device of claim 1, wherein each of the first and second arms (53, 54) includes a plurality of telescoping arm segments (94, 95, 96, 151, 152, 153).

3. The device of claim 1, wherein a distal end portion (92, 147) of each of the first and second arms (53, 54) includes an adjustment mechanism (203, 204) configured to permit pivotal movement of the respective attachment mechanism (251, 261) about a first longitudinal axis of the central post (51).

4. The device of claim 3, wherein the adjustment mechanism (203) of the first arm (53) permits pivotal movement of the corresponding attachment mechanism (251) about an additional axis extending substantially perpendicularly to the first longitudinal axis.

5. The device of claim 1 wherein the first attachment mechanism (251) includes one of a pin, a wire, a screw, a hook and a clamp.

6. The device of claim 5, wherein the second attachment mechanism (261) includes one of a pin, a wire, a screw, a hook and a clamp.

7. The device of claim 1, further comprising:
a third arm (291) having third proximal and distal end portions (292, 293);
a third securement mechanism (301) configured to couple the third proximal end portion (292) of the third arm (291) to a fracture table;
a third attachment mechanism (303) carried by the third distal end portion (293) of the third arm (291) for securing the third arm (291) to a third target portion of a bone, wherein the third arm (291) permits the alignment of the third target portion of a bone with the first and second target portions.

8. The device of claim 3, wherein each of the first and second attachment mechanisms (251, 261) includes an elongate pin having a proximal end and a threaded distal end.

9. The device of claim 8, wherein each of the first and second adjustment mechanisms (203, 204) includes a first element (208) provided with a bore (239) for receiving the proximal end of the respective pin, a second element (207) configured for disposition in juxtaposition with the first element (208) and a pin element (111) for pivotably coupling the first element (208) to the second element (207).

10. The device of claim 9, wherein the pin element (111) is a screw.

11. The device of claim 10, wherein the second element (207) includes one of an additional pin element (219) and a socket (216) for permitting the second element (207) to pivot relative to the distal end portion (92, 147) of the respective arm (53, 54).

## Patentansprüche

1. Vorrichtung zur Knochenfixation, umfassend:
einen ersten Arm (53) mit ersten proximalen und distalen Endabschnitten (91, 92);
einen zweiten Arm (54) mit zweiten proximalen und distalen Endabschnitten (146, 147);
einen ersten Festhaltemechanismus (131, 51), welcher zum Ankuppeln des ersten proximalen Endabschnitts (91) des ersten Arms (53) an einen Behandlungstisch konfiguriert ist;
einen zweiten Festhaltemechanismus (166, 51), welcher zum Ankuppeln des zweiten proximalen Endabschnitts (146) des zweiten Arms (54) an einen Behandlungstisch konfiguriert ist;
einen zentralen Pfosten (51) zum Ankuppeln der ersten und zweiten Festhaltemechanismen (131, 51, 166, 51) an einen Behandlungstisch;
einen ersten Befestigungsmechanismus (251), welcher durch den ersten distalen Endabschnitt (92) des ersten Arms (53) getragen wird und zur Befestigung des ersten Arms (53) an einem ersten Zielabschnitt eines Knochens dient;
einen zweiten Befestigungsmechanismus (261), welcher durch den zweiten distalen Endabschnitt (147) des zweiten Arms (54) getragen wird und zur Befestigung des zweiten Arms (54) an einem zweiten Zielabschnitt eines Knochens dient, wobei
die ersten und zweiten Festhaltemechanismen (131, 51, 166, 51) derart konfiguriert sind, dass sie eine Schwenkbewegung der jeweiligen ersten und zweiten Arme (53, 54) relativ zu dem Behandlungstisch um eine durch den zentralen Pfosten (51) definierte gemeinsame Achse gestatten; und
jeder der ersten und zweiten Arme (53, 54) mehrere Armsegmente (94, 95, 96, 151, 152, 153) umfasst, welche relativ zueinander bewegbar sind, so dass
der erste und zweite Arm (53, 54) derart positionierbar sind, dass eine Ausrichtung der ersten und zweiten Zielabschnitte eines Knochens in Zielpositionen relativ zueinander ermöglicht wird.

2. Vorrichtung nach Anspruch 1, **dadurch gekennzeichnet, dass** jeder der ersten und zweiten Arme (53, 54) mehrere teleskopierbare Armsegmente (94, 95, 96, 151, 152, 153) umfasst.

3. Vorrichtung nach Anspruch 1, **dadurch gekennzeichnet, dass** ein distaler Endabschnitt (92, 147) von jedem der ersten und zweiten Arme (53, 54) einen Einstellmechanismus (203, 204) umfasst, welcher derart konfiguriert ist, dass eine Schwenkbewegung des jeweiligen Befestigungsmechanismus (251, 261) um eine erste Längsachse des zentralen Pfostens (51) ermöglicht wird.

4. Vorrichtung nach Anspruch 3, **dadurch gekennzeichnet, dass** der Einstellmechanismus (203) des ersten Arms (53) eine Schwenkbewegung des korrespondierenden Befestigungsmechanismus (251) um eine zusätzliche Achse ermöglicht, welche sich im Wesentlichen senkrecht zu der ersten Längsachse erstreckt.

5. Vorrichtung nach Anspruch 1, **dadurch gekennzeichnet, dass** der erste Befestigungsmechanismus (251) eines von einem Stift, einem Draht, einer Schraube, einem Haken oder einer Klammer umfasst.

6. Vorrichtung nach Anspruch 5, **dadurch gekennzeichnet, dass** der zweite Befestigungsmechanismus (261) eines von einem Stift, einem Draht, einer Schraube, einem Haken oder einer Klammer umfasst.

7. Vorrichtung nach Anspruch 1, zusätzlich umfassend:
einen dritten Arm (291) mit dritten proximalen und distalen Endabschnitten (292, 293);
einen dritten Festhaltemechanismus (301), welcher zum Ankuppeln des dritten proximalen Endabschnitts (292) des dritten Arms (291) an einen Behandlungstisch konfiguriert ist;
einen dritten Befestigungsmechanismus (303), welcher durch den dritten distalen Endabschnitt (293) des dritten Arms (291) getragen wird und zur Befestigung des dritten Arms (291) an einem dritten Zielabschnitt eines Knochens dient; wobei der dritte Arm (291) eine Ausrichtung des dritten Zielabschnitts eines Knochens zu den ersten und zweiten Zielabschnitten ermöglicht.

8. Vorrichtung nach Anspruch 3, **dadurch gekennzeichnet, dass** jeder der ersten und zweiten Befestigungsmechanismen (251, 261) einen länglichen Stift mit einem proximalen Ende und einem gewindeten distalen Ende umfasst.

9. Vorrichtung nach Anspruch 8, **dadurch gekennzeichnet, dass** jeder der ersten und zweiten Einstellmechanismen (203, 204) ein erstes Element (208) mit einer Bohrung (239) zur Aufnahme des proximalen Endes des jeweiligen Stifts, ein zweites Element (207), welches zur Anordnung neben dem ersten Element (208) konfiguriert ist, und ein Stiftelement (111) zur schwenkbaren Verbindung des ersten Elements (208) mit dem zweiten Element (207) umfasst.

10. Vorrichtung nach Anspruch 9, **dadurch gekennzeichnet, dass** das Stiftelement (111) eine Schraube ist.

11. Vorrichtung nach Anspruch 10, **dadurch gekennzeichnet, dass** das zweite Element (207) eines von einem zusätzlichen Stiftelement (219) und einer Buchse (216) umfasst, um dem zweiten Elements (207) zu ermöglichen, relativ zum distalen Endabschnitt (92, 147) des jeweiligen Arms (53, 54) zu schwenken.

## Revendications

1. Dispositif de fixation d'os, comprenant :
un premier bras (53) ayant des premières portions d'extrémité proximale et distale (91, 92) ;
un second bras (54) ayant des secondes portions d'extrémité proximale et distale (146, 147) ;
un premier mécanisme d'immobilisation (131, 51) configuré pour coupler la première portion d'extrémité proximale (91) du premier bras (53) à une table de fracture ;
un second mécanisme d'immobilisation (166, 51) configuré pour coupler la seconde portion d'extrémité proximale (146) du second bras (54) à la table de fracture ;
un montant central (51) couplant les premier et second mécanismes d'immobilisation (131, 51, 166, 51) à une table de fracture ;
un premier mécanisme de lien (251) porté par la première portion d'extrémité distale (92) du premier bras (53) pour immobiliser le premier bras (53) à une première portion cible d'un os ; et
un second mécanisme de lien (261) porté par la seconde portion d'extrémité distale (147) du second bras (54) pour immobiliser le second bras (54) à une seconde portion cible d'un os,
les premier et second mécanismes d'immobilisation (131, 51, 166, 51) étant configurés pour permettre un mouvement de pivotement des premier et second bras respectifs (53, 54) par rapport à une table de fracture autour d'un axe commun défini par le montant central (51) ; et
chacun desdits premier et second bras (53, 54) incluant une pluralité segments de bras (94, 95, 96, 151, 152, 153) mobile l'un par rapport à l'autre, de sorte que
les premier et second bras (53, 54) sont positionnables de façon à permettre un alignement des première et seconde portions d'os cibles dans des positions cibles l'un par rapport à l'autre.

2. Dispositif selon la revendication 1, dans lequel chacun des premier et second bras (53, 54) inclut une pluralité de segments de bras télescopiques (94, 95, 96, 151, 152, 153).

3. Dispositif selon la revendication 1, dans lequel une portion d'extrémité distale (92, 147) de chacun des premier et second bras (53, 54) inclut un mécanisme d'ajustement (203, 204) configuré pour permettre un mouvement de pivotement du mécanisme de lien respectif (251, 261) autour d'un premier axe longitudinal du montant central (51).

4. Dispositif selon la revendication 3, dans lequel le mécanisme d'ajustement (203) du premier bras (53) permet un mouvement de pivotement du mécanisme de lien correspondant (251) autour d'un axe supplémentaire s'étendant sensiblement perpendiculairement au premier axe longitudinal.

5. Dispositif selon la revendication 1, dans lequel le premier mécanisme de lien (251) inclut un élément parmi une tige, un fil, une vis, un crochet et une pince.

6. Dispositif selon la revendication 5, dans lequel le second mécanisme de lien (261) inclut un élément parmi une tige, un fil, une vis, un crochet et une pince.

7. Dispositif selon la revendication 1, comprenant en outre :
un troisième bras (291) ayant des troisièmes portions d'extrémité proximale et distale (292, 293) ;
un troisième mécanisme d'immobilisation (301) configuré pour coupler la troisième portion d'extrémité proximale (292) du troisième bras (291) à une table de fracture ;
un troisième mécanisme de lien (303) porté par la troisième portion d'extrémité distale (293) du troisième bras (291) pour immobiliser le troisième bras (291) à une troisième portion cible d'un os, le troisième bras (291) permettant l'alignement de la troisième portion cible d'un os avec les première et seconde portions cibles.

8. Dispositif selon la revendication 3, dans lequel chacun des premier et second mécanismes de lien (251, 261) inclut une tige allongée ayant une extrémité proximale et une extrémité distale filetée.

9. Dispositif selon la revendication 8, dans lequel chacun des premier et second mécanismes d'ajustement (203, 204) inclut un premier élément (208) doté d'un alésage (239) destiné à recevoir l'extrémité proximale de la tige respective, un second élément (207) configuré pour une disposition en juxtaposition avec le premier élément (208) et un élément formant tige (111) destiné à coupler à pivotement le premier élément (208) au second élément (207).

10. Dispositif selon la revendication 9, dans lequel l'élément formant tige (111) est une vis.

11. Dispositif selon la revendication 10, dans lequel le second élément (207) inclut l'un parmi un élément supplémentaire formant tige (219) et une alvéole (216) destinée à permettre au second élément (207) de pivoter par rapport à la portion d'extrémité distale (92, 147) du bras respectif (53, 54).
